# EUROPÄISCHE PATENTANMELDUNG

(11) **EP 3 961 212 A1**
(43) Veröffentlichungstag der Anmeldung: **02.03.2022**
(21) Anmeldenummer: 20192536.9
(22) Anmeldetag: 25.08.2020
(51) Int. Cl.: G01N 33/53, B01L 3/02, G01N 1/34

(54) **VORRICHTUNG UND VERFAHREN ZUR ENTFERNUNG VON FREIEM BIOTIN AUS EINER FLÜSSIGEN PROBE**

(71) Anmelder: Siemens Healthcare GmbH, 91052 Erlangen (DE)
(72) Erfinder: Huang, Yiwei, 91058 Erlangen (DE); Mach, Tivadar, 90409 Nürnberg (DE); Paulicka, Peter, 91341 Röttenbach (DE)
(74) Vertreter: Maier, Daniel Oliver

(57) **Zusammenfassung**

Die Erfindung liegt auf dem Gebiet der in vitro Diagnostik und betrifft eine Vorrichtung (1) und ein Verfahren zur Entfernung von freiem Biotin aus einer flüssigen Probe (4). Die Vorrichtung (1) umfasst eine Wandung (2), die einen Hohlraum (3) zur Aufnahme der Probe (4) wenigstens teilweise umschließt, wobei in dem Hohlraum (3) eine Festphase (5) vorgesehen ist, die zur Bindung von freiem Biotin und/oder reversible gebundenem Biotin aus einer Probe (4) mit Bindungspartnern (6) mit Spezifität für Biotin assoziiert ist.

## Beschreibung

Die Erfindung liegt auf dem Gebiet der in vitro Diagnostik und betrifft eine Vorrichtung und ein Verfahren zur Entfernung von freiem Biotin aus einer flüssigen Probe.

Viele in vitro Labortest nutzen Biotintechnologie aufgrund der Fähigkeit von Biotin mit spezifischen Molekülen, wie etwa Proteinen eine Bindung einzugehen, die gemessen werden können, um bestimmte Gesundheitsprobleme zu ermitteln. Beispielsweise wird Biotin genutzt in verschiedenen Hormontests und Test für Marker bezüglich Herzgesundheit, wie z.B. Troponin.

Biotin, auch als Vitamin B7 bezeichnet, ist ein wasserlösliches Vitamin, das in einer Vielzahl von Multivitaminpräparaten und anderen frei verkäuflichen Nahrungsergänzungsmitteln enthalten ist.

Etwa 12 % des Biotins in Blutplasma ist kovalent gebunden, etwa 7 % ist reversible gebunden und etwa 81 % ist freies Biotin, siehe z.B. D. M. Mock, M. I. Malik, "Distribution of biotin in human plasma: most of the biotin is not bound to protein", The American Journal of Clinical Nutrition, Band 56, Ausgabe 2, August 1992, Seiten 427-432.

Insbesondere hohe Konzentrationen von freiem Biotin im Blut von Patienten, z.B. aufgrund von eingenommenen biotinhaltigen Präparaten oder Lebensmitteln, kann dabei signifikant mit bestimmten Labortests interferieren und unrichtige oder verfälschte Testresultate verursachen, die unerkannt bleiben können. Dies führt in der Praxis zu einer Verringerung der Zuverlässigkeit der Labortests, was fatale Folgen für die Gesundheit und das Leben der betroffenen Patienten haben kann. Beispielsweise kann freies Biotin zu Problemen bei verschiedenen etablierten Tests zur Bestimmung von Troponin führen. Dabei kann es insbesondere auch zu verfälscht erniedrigten Werten kommen, was z.B. bei der Diagnose von Herzinfarkten gravierende negative Folgen für Patienten haben kann, wenn z.B. tatsächlich bestehende Herzinfarkte erst verzögert diagnostiziert werden, da verfälscht niedrige Werte von Troponin nicht auf einen Herzinfarkt hinzudeuten scheinen. Je nach z.B. Ernährung und Einnahme von biotinhaltigen Präparaten können Patientenproben z.B. mehr als 100 ng/mL Biotin, oder aber auch mehr als 1200 ng/mL Biotin enthalten.

Die der vorliegenden Erfindung zugrundeliegende Aufgabe bestand also darin, die Zuverlässigkeit von Labortest, die Biotintechnologien nutzen zu erhöhen, insbesondere bei Patienten, die erhöhte Konzentrationen von freiem Biotin im Blut aufweisen.

Es wurde gefunden, dass die Zuverlässigkeit von Labortest, die Biotintechnologien nutzen erhöht werden kann, indem die Konzentration von freiem bzw. reversible gebundenem Biotin in einer Probe reduziert wird mittels einer Festphase, die zur Bindung von freiem Biotin und/oder reversible gebundenem Biotin mit Bindungspartnern mit Spezifität für Biotin assoziiert ist. Dies hat den Vorteil, dass freies und/oder reversible gebundenes Biotin auf besonders einfache und effektive Weise über Bindung an den Bindungspartner mit Spezifität für Biotin der Flüssigkeit der Probe entzogen werden kann. Das so an den Bindungspartner mit Spezifität für Biotin gebundene Biotin verursacht dann bei im Folgenden durchzuführenden Testreaktionen auf Basis von Biotintechnologie keine oder verringerte Interferenzen.

Gegenstand der vorliegenden Erfindung ist eine Vorrichtung zur Aufnahme einer flüssigen Probe, die Vorrichtung umfassend eine Wandung, die einen Hohlraum zur Aufnahme der Probe wenigstens teilweise umschließt, wobei in dem Hohlraum eine Festphase vorgesehen ist, die zur Bindung von freiem Biotin und/oder reversible gebundenem Biotin aus einer Probe mit Bindungspartnern mit Spezifität für Biotin assoziiert ist.

Unter freiem Biotin ist dabei insbesondere Biotin zu verstehen, das weder kovalent noch reversibel gebunden ist. Unter reversibel gebundenem Biotin ist dabei insbesondere Biotin zu verstehen, das weder frei vorliegt noch kovalent gebunden ist. Freies Biotin bezeichnet also insbesondere ein ungebundenes, unkonjugiertes Biotin-Molekül, das beispielsweise weder mit einem Bindungspartner noch mit einer Festphase noch mit einem detektierbaren Label noch mit einer Komponente eines signalbildenden Systems assoziiert ist.

Bevorzugt umfassen die biotin-spezifischen Bindungspartner Avidin, Streptavidin, Tamavidin 2, Shwanavidin und/oder Rhizavidin und/oder ein anderes Biotin-Bindungsprotein. Bevorzugt handelt es sich bei den biotin-spezifischen Bindungspartnern um Peptide. Bevorzugt umfassen dabei die biotin-spezifischen Bindungspartner ein Biotin-Bindungsmotif oder es handelt sich bei ihnen um ein Biotin-Bindungsmotif.

Bevorzugt enthält die Probe vor der erfindungsgemäßen Entfernung von freiem Biotin mehr als 10 ng/mL, bevorzugt mehr als 100 ng/mL, besonders bevorzugt mehr als 1200 ng/mL freies Biotin.

Bevorzugt wird die Konzentration von freiem Biotin in der Probe erfindungsgemäß durch Entfernung von freiem Biotin soweit verringert, dass die Probe nicht mehr als 1200 ng/mL, bevorzugt nicht mehr als 100 ng/mL, besonders bevorzugt nicht mehr als 10 ng/mL Biotin enthält.

Bevorzugt wird die Konzentration von freiem Biotin in der Probe erfindungsgemäß durch Entfernung von freiem Biotin um mindestens 50 Prozent, bevorzugt mindestens 90 Prozent, besonders bevorzugt mindestens 99 Prozent verringert.

Bevorzugt ist eine erfindungsgemäße Vorrichtung ausgewählt aus der Gruppe Blutentnahmeröhrchen, Probengefäß und Pipettenspitze.

Bevorzugt handelt es sich bei einer erfindungsgemäßen Vorrichtung nicht um eine Messküvette, da andernfalls Testreagenzien auch an z.B. die entsprechend beschichteten Oberflächen der Messküvette binden würden. Dies könnte zu fehlerhaften Messergebnissen führen und sollte daher vermieden werden.

Bevorzugt ist die Festphase, die zur Bindung von freiem Biotin mit Bindungspartnern mit Spezifität für Biotin assoziiert ist, die Wandung oder zumindest ein Teilbereich der Wandung der Vorrichtung. Beispielsweise ist an der inneren Wand des Blutentnahmeröhrchens, Probengefäßes und/oder der Pipettenspitze eine Kunststoffbeschichtung, beispielsweise polymerisierter Latex, aufgebracht. An die Kunststoffbeschichtung ist kovalent z.B. Avidin und/oder Streptavidin gebunden.

Bevorzugt ist die Festphase, die zur Bindung von freiem Biotin mit Bindungspartnern mit Spezifität für Biotin assoziiert ist, eine partikuläre Festphase.

Bevorzugt umfasst die partikuläre Festphase Teilchen, die einen ungefähren Durchmesser von wenigstens 20 nm und nicht mehr als 1000 µm aufweisen. Besonders bevorzugt sind dabei größere Teilchen mit einem Durchmesser von wenigstens einem Mikrometer, ganz besonders bevorzugt von wenigstens 10 Mikrometern. Bevorzugt kann dabei Biotin in die Teilchen eintreten. Die vergleichsweise große Größe der Mikropartikel hat den Vorteil, dass sich die Teilchen im Schwerefeld der Erde besser im unteren Bereich der Vorrichtung absetzten und z.B. weniger leicht aus der Vorrichtung heraus pipettiert werden.

Bevorzugt weist der Hohlraum einen ersten Filter auf, der einen ersten und einen zweiten Bereich innerhalb des Hohlraums voneinander abtrennt, wobei der Filter wenigstens teilweise, bevorzugt vollständig, für die Probe durchlässig und für die partikuläre Festphase undurchlässig ist, und wobei die partikuläre Festphase, die mit Bindungspartnern mit Spezifität für Biotin assoziiert ist, in dem ersten Bereich des Hohlraums vorgesehen ist und wobei der zweite Bereich frei ist von Bindungspartnern mit Spezifität für Biotin.

Bevorzugt ist der Filter dabei so ausgestaltet, dass die Flüssigkeit, z.B. Blutplasma, durch ihn möglichst ungehindert hindurchfließen kann und gleichzeitig die Teilchen der partikulären Festphase nicht durch den Filter hindurchtreten können und zurückgehalten werden, wenn z.B. aus der Vorrichtung Flüssigkeit heraus pipettiert wird. Dabei sind die Poren des Filters vorteilhafterweise möglichst groß, um die Flüssigkeit möglichst ungehindert hindurchzulassen bzw. eine möglichst große Diffusion der Flüssigkeit durch den Filter zu erreichen, jedoch kleiner als die Teilchen, die zurückgehalten werden sollen. Der Filter kann dabei aus verschiedensten geeigneten Materialien aufgebaut sein. Auch der detaillierte Aufbau des Filters kann dabei stark variieren.

Bevorzugt weist der Hohlraum einen ersten und einen zweiten Filter auf, wobei der erste Filter und der zweite Filter einen ersten Bereich innerhalb des Hohlraums abtrennen, wobei die Filter jeweils wenigstens teilweise, bevorzugt vollständig, für die Probe durchlässig und für die partikuläre Festphase undurchlässig sind und wobei die partikuläre Festphase, die mit Bindungspartnern mit Spezifität für Biotin assoziiert ist, in dem ersten Bereich des Hohlraums vorgesehen ist und wobei alle anderen Bereiche des Hohlraums frei sind von Bindungspartnern mit Spezifität für Biotin.

Bevorzugt ist der erste und/oder der zweite Filter entsprechend dem Filter wie oben beschrieben ausgestaltet.

Ein weiterer Gegenstand der Erfindung ist eine Verwendung einer erfindungsgemäßen Vorrichtung zur Entfernung von freiem Biotin aus einer flüssigen Probe, vorzugsweise aus einer humanen oder tierischen Körperflüssigkeitsprobe, besonders bevorzugt aus einer humanen oder tierischen Körperflüssigkeitsprobe aus der Gruppe Vollblut, Plasma und Serum.

Ein weiterer Gegenstand der Erfindung ist ein Verfahren zum Entfernen von freiem Biotin aus einer Probe, das Verfahren aufweisend den folgenden Schritt:
- Verbringen der Probe in einen Hohlraum einer Vorrichtung, wobei in dem Hohlraum eine Festphase vorgesehen ist, die zur Bindung von freiem Biotin aus der Probe mit Bindungspartnern mit Spezifität für Biotin assoziiert ist.

Bevorzugt erfolgt eine Pipettierung der flüssigen Probe in den Hohlraum der Vorrichtung hinein und/oder aus diesem heraus mittels eines automatischen Pipettors umfassend eine Pipettenspitze und/oder eine Pipettiernadel. Dabei wird vorteilshafterweise eine Eintauchtiefe der Pipettenspitze bzw. der Pipettiernadel und/oder eine Pipettiergeschwindigkeit so gewählt, dass die Festphase mit Bindungspartnern mit Spezifität für Biotin in dem Hohlraum verbleibt und nicht heraus pipettiert wird.

Bevorzugt wird die Probe durch Ansaugen in einen Bereich des Hohlraums einer Pipettenspitze verbracht, welcher Bereich durch ein oder mehrere Filter abgetrennt ist und in dem eine partikuläre Festphase, die zur Bindung von freiem Biotin aus der Probe mit Bindungspartnern mit Spezifität für Biotin assoziiert ist, vorgesehen ist, wobei der oder die Filter jeweils wenigstens teilweise, bevorzugt vollständig, für die Probe durchlässig und für die partikuläre Festphase undurchlässig sind. Bevorzugt erfolgt das Ansaugen so, dass der eine Filter oder die mehreren Filter wenigstens teilweise, bevorzugt vollständig intakt bleibt bzw. bleiben und es insbesondere nicht zu einem Durchbruch oder mehreren Durchbrüchen kommt.

Bevorzugt wird die Probe in einen Hohlraum einer Vorrichtung verbracht und dort inkubiert, welcher Hohlraum von einer Wandung wenigstens teilweise umschlossen wird, und wobei die Wandung oder zumindest ein Teilbereich der Wandung die Festphase ist, die zur Bindung von freiem Biotin mit Bindungspartnern mit Spezifität für Biotin assoziiert ist. Bevorzugt wird die Dauer der Inkubation so gewählt, dass das freie Biotin und/oder reversible gebundene Biotin in der Probe wenigstens teilweise, bevorzugt vollständig durch die Bindungspartner mit Spezifität für Biotin der Festphase gebunden wird. Beispielsweise wird dies dadurch erreicht, dass eine Steuerung des Pipettors so erfolgt, dass der Zeitabstand zwischen Verbringung in den Hohlraum hinein und der Verbringung aus dem Hohlraum heraus entsprechend ausreichend groß gewählt wird.

Bevorzugt ist die in dem Hohlraum vorgesehene Festphase, die zur Bindung von freiem Biotin mit Bindungspartnern mit Spezifität für Biotin assoziiert ist, eine partikuläre Festphase und wobei das Verfahren weiter die folgenden Schritte umfasst:
- Inkubation der Probe, wobei die Probe während der Inkubation wenigstens teilweise mit der partikulären Festphase in Kontakt steht,
- Trennung der Probe und der partikulären Festphase, bevorzugt mittels Ausübung einer Zentrifugalkraft auf die Vorrichtung enthaltend die Probe.

Bevorzugt umfasst ein erfindungsgemäßes Verfahren eine Verdünnung der Probe. Bevorzugt wird freies Biotin mittels eines Verdünnungsschritts, z.B. mit einer erfindungsgemäßen Pipettierspitze, aus der Probe entfernt.

Bevorzugt ist das erfindungsgemäße Verfahren Bestandteil eines Verfahrens zur Verdünnung der Probe, wobei die Entfernung des freien Biotins aus der Probe bevorzugt während eines Verdünnungsschritts oder mehrerer Verdünnungsschritte der Probe oder von Teilen der Probe erfolgt. Dies hat den Vorteil, dass nicht länger ein zusätzlicher Zeitaufwand für die Entfernung des freien Biotins benötigt wird und die automatische Analyse der Probe zuverlässiger und gleichzeitig beschleunigt erfolgen kann.

Bevorzugt ist die Probe eine humane oder tierische Körperflüssigkeitsprobe, bevorzugt eine humane oder tierische Körperflüssigkeitsprobe aus der Gruppe Vollblut, Plasma und Serum.

Ein weiterer Gegenstand der Erfindung ist ein Analyzer zum automatischen Analysieren einer Probe, wobei der Analyzer ein Steuergerät umfasst, das so konfiguriert ist, dass der Analyzer ein erfindungsgemäßes Verfahren durchführen kann und/oder wobei der Analyzer eine erfindungsgemäße Vorrichtung umfasst.

Bevorzugt ist vorgesehen, dass der Analyzer freies Biotin nur aus Proben oder Teilen von Proben entfernt, an denen vorbestimmte Analysen durchgeführt werden sollen, die sensitiv auf freies Biotin in der Probe reagieren. Bevorzugt ist dazu vorgesehen, dass der Analyzer eine erfindungsgemäße Vorrichtung und/oder ein erfindungsgemäßes Verfahren nur in solchen Fällen einsetzt. So werden z.B. erfindungsgemäße Pipettenspitzen oder Gefäße verwendet, wenn freies Biotin aus der Probe entfernt werden soll, wohingegen gewöhnliche Pipettenspitzen oder Gefäße verwendet werden, wenn die Entfernung von freiem Biotin für die vorbestimmten Analysen nicht notwendig ist.

Unter dem Begriff "partikuläre Festphase" sind im Sinne dieser Erfindung nicht-zelluläre Teilchen zu verstehen. Die nicht-zelluläre Teilchen weisen dabei beispielsweise einen ungefähren Durchmesser von wenigstens 20 nm und nicht mehr als 5000 µm auf. Die Mikropartikel können regelmäßig oder unregelmäßig geformt sein. Sie können Kugeln, Sphäroide, Kugeln mit mehr oder weniger großen Kavitäten oder Poren darstellen. Die Mikropartikel können aus organischem, aus anorganischem Material oder aus einer Mischung oder einer Kombination von beiden bestehen. Sie können aus einem porösen oder nicht porösen, einem schwellfähigen oder nicht schwellfähigen Material bestehen. Prinzipiell können die Mikropartikel jegliche Dichte haben, bevorzugt sind jedoch Partikel mit einer Dichte, die der Dichte des Wassers nahe kommt, wie etwa 0,7 bis etwa 1,5 g/ml. Die bevorzugten Mikropartikel sind in wässrigen Lösungen suspendierbar und möglichst lange suspensionsstabil. Sie mögen durchsichtig, teilweise durchsichtig oder undurchsichtig sein. Die Mikropartikel können aus mehreren Schichten bestehen wie beispielsweise die sogenannten "core-and-shell" Partikel mit einem Kern und einer oder mehreren umhüllenden Schichten. Der Begriff Mikropartikel umfasst beispielsweise Farbstoffkristalle, Metallsolen, Silica-Partikel, Glaspartikel und Magnetpartikel. Bevorzugte Mikropartikel sind in wässrigen Lösungen suspendierbare und aus wasserunlöslichem Polymermaterial bestehende Partikel, insbesondere aus substituierten Polyethylenen. Ganz besonders bevorzugt sind Latexpartikel z.B. aus Polystyrol, Acrylsäurepolymeren, Methacrylsäurepolymeren, Acrylnitril-Polymeren, Acrylnitril-Butadien-Styrol, Polyvinylacetat-Acrylat, Polyvinylpyridin, Vinylchlorid-Acrylat. Von besonderem Interesse sind Latexpartikel mit reaktiven Gruppen an ihrer Oberfläche wie beispielsweise Carboxyl-, Amino- oder Aldehydgruppen, die eine kovalente Bindung eines anti-TNFalpha-Bindungspartners an die Latexpartikel erlauben. Humane, tierische, pflanzliche oder pilzliche Zellen oder Bakterien sind im Sinne dieser Erfindung explizit nicht von dem Begriff "partikuläre Festphase" umfasst.

Der Begriff "assoziiert" ist breit zu verstehen und umfasst beispielsweise eine kovalente und eine nicht-kovalente Bindung, eine direkte und eine indirekte Bindung, die Adsorption an eine Oberfläche und den Einschluss in eine Vertiefung oder einen Hohlraum etc. Bei einer kovalenten Bindung sind die Antikörper über eine chemische Bindung an die Festphase gebunden. Beispiele für eine nicht-kovalente Bindung sind die Oberflächenadsorption, der Einschluss in Hohlräume oder die Bindung von zwei spezifischen Bindungspartnern. Neben einer direkten Bindung an die Festphase können die Antikörper auch indirekt über spezifische Wechselwirkung mit anderen spezifischen Bindungspartnern an die Festphase gebunden sein.

Die Begriffe Avidin, Streptavidin, Tamavidin 2, Shwanavidin und Rhizavidin umfassen bevorzugt nicht nur das jeweilige vollständige Molekül, sondern auch Fragmente des vollständigen Moleküls, an die Biotin entsprechend binden kann. Die Begriffe umfassen ferner bevorzugt nicht nur die wildtypischen Moleküle oder Fragmente davon, sondern auch Varianten mit einer oder mehr Aminosäuresubstitutionen, an die Biotin binden kann.

Die Erfindung wird anhand von Zeichnungen exemplarisch näher erläutert. Dabei dienen die folgenden Beispiele der Veranschaulichung der vorliegenden Erfindung und sind nicht als Einschränkung zu verstehen. Gleiche Teile sind in allen Figuren mit denselben Bezugszeichen versehen.

Die Figuren zeigen:

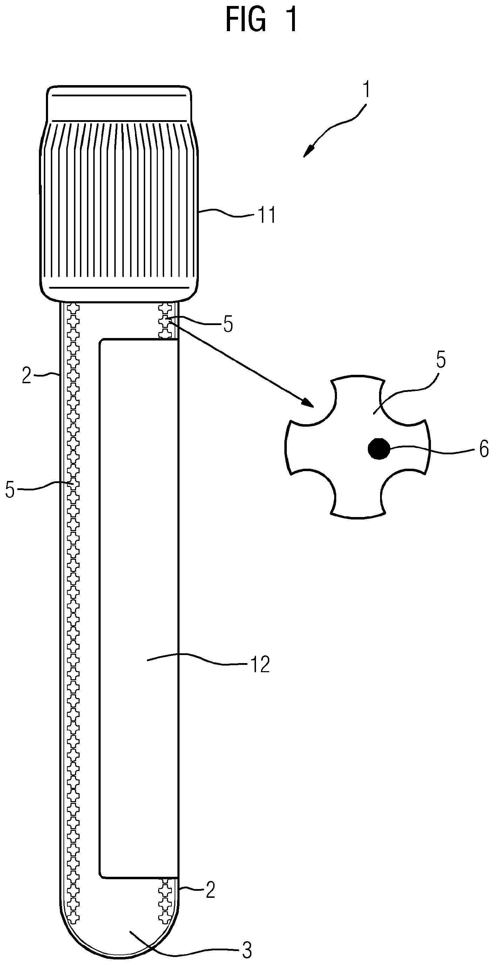

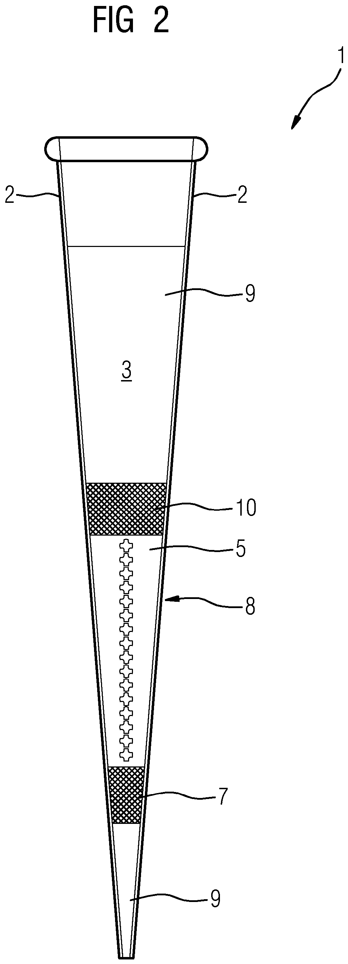

**FIG 1** **und** **FIG 2** **schematisch den Aufbau einer Vorrichtung (1) zur Aufnahme einer flüssigen Probe (4).**

Die Vorrichtung (1) gemäß FIG 1 und FIG 2 kann in einem nicht näher dargestelltes Analysegerät, welches zur Ausführung einer Vielzahl von Analysen von Proben ausgestaltet ist, automatisch prozessiert werden. Dazu umfasst das automatische Analysegerät eine Vielzahl von nicht gezeigten Pipettiereinrichtungen und Transporteinrichtungen sowie weiterhin eine Steuereinheit zum automatisierten Steuern eines erfindungsgemäßen Verfahrens zum Entfernen von freiem Biotin aus einer Probe (4) in dem automatischen Analysegerät mittels einer erfindungsgemäßen Vorrichtung (1).

FIG 1 zeigt eine Vorrichtung (1) zur Aufnahme einer flüssigen Probe (4), wobei die Vorrichtung (1) ein zylindrisch geformtes Blutentnahmeröhrchen mit einem runden Querschnitt ist. Das Blutentnahmeröhrchen kann z.B. aus Glas und/oder Kunststoff bestehen. Das Blutentnahmeröhrchen umfasst eine Wandung (2), die einen Hohlraum (3) zur Aufnahme der Probe (4) teilweise umschließt. Das Blutentnahmeröhrchen weist eine Öffnung auf (verdeckt), die mit einem Verschluss (11) verschlossen ist. In dem Hohlraum (3) ist eine Festphase (5) vorgesehen, die zur Bindung von freiem Biotin und/oder reversible gebundenem Biotin aus einer Probe (4) mit Bindungspartnern (6) mit Spezifität für Biotin assoziiert ist. Die Wandung (2) umfasst dabei die Festphase (5) zumindest in einem Teilbereich. Z.B. ist die Festphase (5) an der gesamten Innenseite der Wandung (2) oder in einem Teilbereich der Innenseite der Wandung (2) angeordnet. Die Innenseite der Wandung (2) ist dabei die Seite der Wandung (2), die in Richtung des Hohlraums (3) orientiert ist. Die flüssige Probe (4) kann z.B. in die Vorrichtung (1) durch die Öffnung (10) verbracht werden. Dazu kann der Verschluss (11) entfernt werden. Alternativ kann auch die Probe (4) mittels einer hierfür ausgelegten Nadel oder Pipettiernadel, die den Verschluss (10) durchstechen kann, durch den Verschluss (10) hindurch in die Vorrichtung (1) verbracht werden. Dies kann z.B. direkt schon während des Blutabnahmevorgangs geschehen. Bei den biotin-spezifischen Bindungspartnern (6) handelt es sich z.B. Avidin und/oder Streptavidin und die Festphase (5) umfasst eine Avidin- und/oder Streptavidin-Matrix.

FIG 2 zeigt eine Vorrichtung (1) zur Aufnahme einer flüssigen Probe (4), wobei die Vorrichtung (1) eine Pipettenspitze ist. Die Pipettenspitze weist eine um eine Längsachse rotationssymmetrische Form auf, die ähnlich eines geraden Kreiskegels ist. Im Bereich der gedachten Spitze des Kreiskegels befindet sich eine erste Öffnung und im Bereich mit dem größten Radius des Kreiskegels eine zweite Öffnung. Im Bereich dieser zweiten Öffnung kann die Pipettenspitze z.B. auf einen von Hand betriebenen Pipettor oder einen automatischen Pipettor aufgesteckt werden. Die Pipettenspitze umfasst eine Wandung (2), die einen Hohlraum (3) zur Aufnahme der Probe (4) teilweise umschließt. Durch die erste Öffnung kann dann die flüssige Probe (4) hindurch in den Hohlraum (3) verbrachte werden, z.B. durch Ansaugen mittels des Pipettors. In dem Hohlraum (3) ist eine Festphase (5) vorgesehen, die zur Bindung von freiem Biotin und/oder reversible gebundenem Biotin aus einer Probe (4) mit Bindungspartnern (6) mit Spezifität für Biotin assoziiert ist. Der Hohlraum (3) weist einen ersten (7) und einen zweiten Filter (10) auf, wobei der erste Filter (7) und der zweite Filter (10) einen ersten Bereich (8) innerhalb des Hohlraums (3) abtrennen. Die Filter (7, 10) sind jeweils wenigstens teilweise, bevorzugt vollständig, für die Probe (4) durchlässig und für die partikuläre Festphase (5) undurchlässig. Die partikuläre Festphase (5), die mit Bindungspartnern (6) mit Spezifität für Biotin assoziiert ist, befindet sich in dem ersten Bereich (8) des Hohlraums (3). Alle anderen Bereiche des Hohlraums (3) sind frei von Bindungspartnern (6) mit Spezifität für Biotin. Die von mit Bindungspartnern (6) mit Spezifität für Biotin freien Bereiche umfassen insbesondere einen zweiten Bereich (9), der den Bereich der ersten Öffnung (Spitze) und den Beriech der zweiten Öffnung (Bereich mit dem größten Radius des Kreiskegels) umfasst.

### BEZUGSZEICHENLISTE

- 1: Vorrichtung
- 2: Wandung
- 3: Hohlraum
- 4: Probe
- 5: Festphase
- 6: Bindungspartner
- 7: erster Filter
- 8: erster Bereich
- 9: zweiter Bereich
- 10: zweiter Filter
- 11: Verschluss
- 12: Label

## Patentansprüche

1. Vorrichtung (1) zur Aufnahme einer flüssigen Probe (4),
die Vorrichtung (1) umfassend eine Wandung (2), die einen Hohlraum (3) zur Aufnahme der Probe (4) wenigstens teilweise umschließt,
**dadurch gekennzeichnet, dass**
in dem Hohlraum (3) eine Festphase (5) vorgesehen ist, die zur Bindung von freiem Biotin und/oder reversible gebundenem Biotin aus einer Probe (4) mit Bindungspartnern (6) mit Spezifität für Biotin assoziiert ist.

2. Vorrichtung (1) nach Anspruch 1, wobei die Biotin-spezifischen Bindungspartner (6) Avidin, Streptavidin, Tamavidin 2, Shwanavidin und/oder Rhizavidin und/oder ein anderes Biotin-Bindungsprotein (Peptid) umfassen.

3. Vorrichtung (1) nach einem der vorhergehenden Ansprüche und ausgewählt aus der Gruppe Blutentnahmeröhrchen, Probengefäß und Pipettenspitze.

4. Vorrichtung (1) nach einem der vorhergehenden Ansprüche, wobei die Festphase (5), die zur Bindung von freiem Biotin mit Bindungspartnern (6) mit Spezifität für Biotin assoziiert ist, die Wandung (2) oder zumindest ein Teilbereich der Wandung (2) der Vorrichtung (1) ist.

5. Vorrichtung (1) nach einem der Ansprüche 1 bis 3, wobei die Festphase (5), die zur Bindung von freiem Biotin mit Bindungspartnern (6) mit Spezifität für Biotin assoziiert ist, eine partikuläre Festphase (5) ist.

6. Vorrichtung (1) nach Anspruch 5, wobei der Hohlraum (3) einen ersten Filter (7) aufweist, der einen ersten (8) und einen zweiten Bereich (9) innerhalb des Hohlraums (3) voneinander abtrennt, wobei der erste Filter (7) wenigstens teilweise, bevorzugt vollständig, für die Probe (4) durchlässig und für die partikuläre Festphase (5) undurchlässig ist, und wobei die partikuläre Festphase (5), die mit Bindungspartnern (6) mit Spezifität für Biotin assoziiert ist, in dem ersten Bereich (8) des Hohlraums vorgesehen ist und wobei der zweite Bereich (9) frei ist von Bindungspartnern mit Spezifität für Biotin.

7. Vorrichtung (1) nach Anspruch 5, wobei der Hohlraum (3) einen ersten (7) und einen zweiten Filter (10) aufweist, wobei der erste Filter (7) und der zweite Filter (10) einen ersten Bereich (8) innerhalb des Hohlraums (3) abtrennen, wobei die Filter (7, 10) jeweils wenigstens teilweise, bevorzugt vollständig, für die Probe (4) durchlässig und für die partikuläre Festphase (5) undurchlässig sind und wobei die partikuläre Festphase (5), die mit Bindungspartnern (6) mit Spezifität für Biotin assoziiert ist, in dem ersten Bereich (8) des Hohlraums (3) vorgesehen ist und wobei alle anderen Bereiche des Hohlraums (3) frei sind von Bindungspartnern (6) mit Spezifität für Biotin.

8. Verwendung einer Vorrichtung (1) gemäß einem der vorhergehende Ansprüche zur Entfernung von freiem Biotin aus einer flüssigen Probe (4), vorzugsweise aus einer humanen oder tierischen Körperflüssigkeitsprobe, besonders bevorzugt aus einer humanen oder tierischen Körperflüssigkeitsprobe aus der Gruppe Vollblut, Plasma und Serum.

9. Verfahren zum Entfernen von freiem Biotin aus einer Probe (4), das Verfahren aufweisend den folgenden Schritt:
- Verbringen der Probe (4) in einen Hohlraum (3) einer Vorrichtung (1), wobei in dem Hohlraum (3) eine Festphase (5) vorgesehen ist, die zur Bindung von freiem Biotin aus der Probe (4) mit Bindungspartnern (6) mit Spezifität für Biotin assoziiert ist.

10. Verfahren nach Anspruch 9, wobei die Probe (4) durch Ansaugen in einen Bereich des Hohlraums (3) einer Pipettenspitze verbracht wird, welcher Bereich durch ein oder mehrere Filter (7, 10) abgetrennt ist und in dem eine partikuläre Festphase (5), die zur Bindung von freiem Biotin aus der Probe (4) mit Bindungspartnern (6) mit Spezifität für Biotin assoziiert ist, vorgesehen ist, wobei der oder die Filter (7, 10) jeweils wenigstens teilweise, bevorzugt vollständig, für die Probe (4) durchlässig und für die partikuläre Festphase (5) undurchlässig sind.

11. Verfahren nach Anspruch 9, wobei die Probe (4) in einen Hohlraum (3) einer Vorrichtung (1) verbracht und dort inkubiert wird, welcher Hohlraum (3) von einer Wandung (2) wenigstens teilweise umschlossen wird, und wobei die Wandung (2) oder zumindest ein Teilbereich der Wandung (2) die Festphase (5) ist, die zur Bindung von freiem Biotin mit Bindungspartnern (6) mit Spezifität für Biotin assoziiert ist.

12. Verfahren nach Anspruch 9, wobei die in dem Hohlraum (3) vorgesehene Festphase (5), die zur Bindung von freiem Biotin mit Bindungspartnern (6) mit Spezifität für Biotin assoziiert ist, eine partikuläre Festphase (5) ist und wobei das Verfahren weiter die folgenden Schritte umfasst:
- Inkubation der Probe (4), wobei die Probe (4) während der Inkubation wenigstens teilweise mit der partikulären Festphase (5) in Kontakt steht,
- Trennung der Probe (4) und der partikulären Festphase (5) mittels Ausübung einer Zentrifugalkraft auf die Vorrichtung (1) enthaltend die Probe (4).

13. Verfahren nach einem der Ansprüche 9 bis 12, wobei die Probe (4) eine humane oder tierische Körperflüssigkeitsprobe, bevorzugt eine humane oder tierische Körperflüssigkeitsprobe aus der Gruppe Vollblut, Plasma und Serum ist.

14. Verfahren nach einem der Ansprüche 9 bis 13, wobei das Verfahren Bestandteil eines Verfahrens zur Verdünnung der Probe (4) ist, wobei die Entfernung des freien Biotins aus der Probe (4) bevorzugt während eines Verdünnungsschritts oder mehrerer Verdünnungsschritte der Probe (4) erfolgt.

15. Analyzer zum automatischen Analysieren einer Probe (4), wobei der Analyzer ein Steuergerät umfasst, das so konfiguriert ist, dass der Analyzer ein Verfahren nach einem der Ansprüche 9 bis 14 durchführen kann und/oder wobei der Analyzer eine Vorrichtung (1) nach einem der Ansprüche 1 bis 7 umfasst.
